(19) Europäisches Patentamt
European Patent Office
Office européen des brevets

(11) Publication number: **0 214 743 B1**

(12) **EUROPEAN PATENT SPECIFICATION**

(45) Date of publication of patent specification: **12.05.93** (51) Int. Cl.$^5$: **A61K 31/47**

(21) Application number: **86305804.6**

(22) Date of filing: **29.07.86**

(54) **Use of quinoline derivatives in the manufacture of medicaments for the treatment of cerebrovascular disorders and cerebral senility.**

(30) Priority: **31.07.85 GB 8519306**
**13.11.85 GB 8527994**

(43) Date of publication of application:
**18.03.87 Bulletin 87/12**

(45) Publication of the grant of the patent:
**12.05.93 Bulletin 93/19**

(84) Designated Contracting States:
**BE CH DE FR GB IT LI LU NL SE**

(56) References cited:
**EP-A- 0 093 535**
**WO-A-85/00602**

(73) Proprietor: **BEECHAM GROUP PLC**
**Beecham House Great West Road**
**Brentford Middlesex TW8 9BD(GB)**

Proprietor: **BEECHAM - WUELFING GmbH & Co. KG**
**Stresemannallee 6 P.O. Box 25**
**W-4040 Neuss(DE)**

(72) Inventor: **Stemp, Geoffrey, c/o Beecham Pharmaceuticals**
**Med. Research Center, Coldharbour Road**
**The Pinnacles, Harlow, Essex CM19 5AD(GB)**
Inventor: **Evans, John Morris, c/o Beecham Pharmaceuticals**
**Med. Research Center, Coldharbour Road**
**The Pinnacles, Harlow, Essex CM19 5AD(GB)**
Inventor: **Nicholson, Charles David**
**Beecham-Wuelfing GmbH & Co.KG, Postfach 4**
**W-3212 Gronau (Leine)(DE)**
Inventor: **Angersbach, Dieter**
**Beecham-Wuelfing GmbH & Co.KG, Postfach 4**
**W-3212 Gronau (Leine)(DE)**

(74) Representative: **Valentine, Jill Barbara**
**Beecham Pharmaceuticals Patents & Trade Marks Dept. Great Burgh Yew Tree Bottom Road**
**Epsom Surrey KT18 5XO (GB)**

**EP 0 214 743 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the use of a compound of formula I for the manufacture of a medicament for the treatment and/or prophylaxis of cerebrovascular disorders and/or disorders associated with cerebral senility.

PCT Patent publication No. WO85/00602 discloses compounds of formula (I):

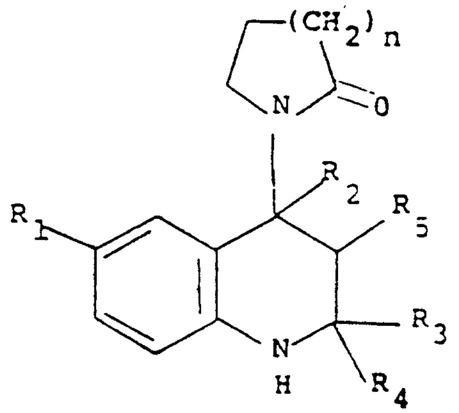

(I)

wherein:

n is 1 or 2;

$R_1$ is chloro, bromo, $C_{1-6}$alkylcarbonyl or cyano;

one of $R_3$ and $R_4$ is hydrogen or $C_{1-4}$ alkyl and the other is $C_{1-4}$ alkyl or $R_3$ and $R_4$ together are $C_{2-5}$ polymethylene;

and either

$R_5$ is hydroxy, $C_{1-3}$ alkoxy or $C_{1-8}$ acyloxy; and

$R_2$ is hydrogen and the cyclic amide moiety and $R_5$ are trans;

or $R_5$ and $R_2$ together form a bond.

The compounds of the formula (I) are described as having blood-pressure lowering activity, making them of use in the treatment of hypertension.

It has now been discovered that the compounds of formula (I) also have a protective effect against the consequences of cerebral metabolic inhibition and/or increase the oxygen tension of the normoxic and ischaemic cortex. The compounds are therefore of potential use in the treatment of cerebrovascular disorders and disorders associated with cerebral senility in mammals including humans.

Accordingly, the present invention provides the use of a compound of formula I for the manufacture of a medicament for the treatment and/or prophylaxis of cerebrovascular disorders and/or disorders associated with cerebral senility in mammals, such as humans, which comprises administering to the mammal in need of such treatment and/or prophylaxis an effective and/or prophylactic amount of a compound of formula (I) as hereinbefore defined.

The administration to the mammal may be by way of oral administration or parenteral administration.

An amount effective to treat the disorders hereinbefore described depends on the usual factors such as the nature and severity of the disorders being treated and the weight of the mammal. However, a unit dose will normally contain 0.1 to 100 mg for example 0.5 to 50 mg, of the compound of the invention. Unit doses will normally be administered once or more than once a day, for example 2, 3, 4, 5 or 6 times a day, more usually 1 to 4 times a day, such that the total daily dose is normally in the range, for a 70 kg adult of 0.1 to 250 mg, for example 1 to 150 mg,that is in the range of approximately 0.002 to 3.5 mg/kg/day, more usually 0.02 to 3 mg/kg/day, for example 0.7 to 2 mg/kg/day.

It is greatly preferred that the compound of formula (I) is administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more

convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing.
Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

In addition such compositions may contain further active agents such as anti-hypertensive agents and diuretics.

As is common practice, the compositions will usually be accompanied by written or printed directions for use in the medical treatment concerned.

The present invention also provides a compound of formula (I) for use in the treatment and/or prophylaxis of cerebrovascular disorders and/or disorders associated with cerebral senility. Such treatment and/or prophylaxis may be carried out as hereinbefore described.

In preferred compounds of formula (I), n is often 1.

Suitable values for $R_1$ include chloro, bromo, acetyl, propionyl, n-, and iso-butyryl and cyano. Often $R_1$ is chloro, bromo, acetyl or cyano.

The alkyl groups or alkyl moieties of alkyl-containing groups for $R_1$ are, preferably, methyl or ethyl.

Favourably, $R_3$ and $R_4$ are both alkyl having from 1 to 4 carbon atoms including methyl, ethyl or n-propyl. In particular, they are both methyl or ethyl, preferably both methyl.

Suitable examples for $R_5$ when $C_{1-3}$ alkyloxy include methoxy, ethoxy, n- and iso-propyoxy, preferably methoxy.

Suitable examples for $R_5$ when acyloxy include carboxylic acyloxy such as acetoxy, propionyloxy and benzoyloxy.

There is a group of compounds within formula (I) of formula (II):

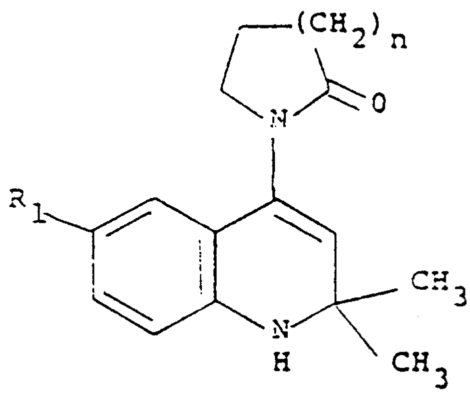

(II)

wherein variables are as defined in formula (I).

Suitable and preferred values for the variables are as described for the corresponding variables under formula (I).

It will be appreciated that there is another sub-group of compounds within formula (I) of formula (III):

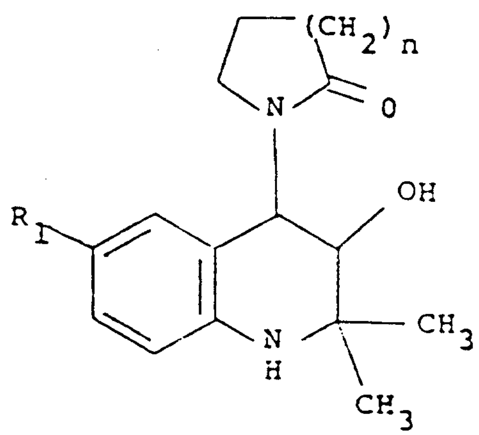

(III)

wherein the variables are as defined in formula (I).

A preferred compound of formula(I) is trans-2,2-dimethyl-6-cyano-4-(2-oxo-1-pyrrolidinyl)-1,2,3,4-tetrahydroquinolin-3-ol (Compound A).

Two other compounds of formula (I) are 6-bromo-2,2-dimethyl-4-(2-oxo-1-pyrrolidinyl)-1, 2-dihydroquinoline (Compound B) and 6-cyano-2,2-dimethyl-4-(2-oxo-1-pyrrolidinyl)-1,2-dihy-droqu inoline (Compound C), which may be prepared as described hereinafter.

The compounds used in the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

Other suitable compounds are those exemplified in WO-A-85/00602 i.e.
trans-2,2-dimethyl-6-chloro-4-(2-oxo-1-pyrrolidinyl)-1,2,3,4,-tetrahydroquinolin-3-ol;
6-chloro-2,2-dimethyl-4-(2-oxo-1-pyrrolidinyl)-1,2-dihydroquinoline;
trans-2,2-dimethyl-6-bromo-4-(2-oxo-1-pyrrolidinyl)-1,2, 3,4-tetrahydroquinolin-3-ol.

In the preparations described hereinafter, Compounds B and C are prepared via the method of Example 3 of WO-A-85/00602. However compounds B and C may alternatively be prepared from the novel intermediate <u>cis</u> -2,2-dimethyl-6-bromo-4-(2-oxo-1-pyrrolidinyl)-1,2,3, 4-tetrahydroquinolin-3-ol, which is obtained as a side product in the aforesaid Example 3.

The invention provides the use of a compound of formula (I) for the manufacture of a medicament for the treatment or prophylaxis of cerebrovascular disorders and/or disorders associated with cerebral senility.

Such compositions may be prepared in the manner as hereinbefore described.

The following pharmacological data illustrate the activity of a compound of formula (I) in tests which are indicative of compounds of potential use in the treatment of cerebrovascular disorders and disorders associated with cerebral senility in mammals.

Pharmacological Data

1. Triethyltin – induced cerebral oedema in the rat.

The cerebral oedema is induced by oral administrations repeated for 5 consecutive days – one administration per day – of triethyltin chloride at a dose of 2 mg/kg. The test compound is also administered orally twice daily as aqueous solution or suspension (1ml/100g body – weight); these administrations are given during the 5 days of intoxication with tin. Three groups of 10 male specific pathogen – free (SPF) Wistar rats of 280 ± 10g body – weight are used:

- 1 control group
- 1 group intoxicated with triethyltin
- 1 group intoxicated with triethyltin and treated with the studied compound.

The rats are killed on the evening of the fifth day; the brain is removed, weighed fresh and after desiccation to constant weight and the water content of each brain is calculated:

$[H_2O]$ = fresh weight – dry weight.

The following are then calculated:

- the mean water content (M ± Sm%) of each group;
- the protection index P due to the administered compound:

$$P\% = 1 - \frac{[H_2O] \text{ treated group} - [H_2O] \text{ control group}}{[H_2O] \text{triethyltin group} - [H_2O] \text{ control group}} \times 100$$

The significance is evaluated by the Mann – Whitney test.

The results are shown in Table 1.

TABLE 1

| Compound | Dosage mg/kg p.o. | % inhibition of cerebral oedema formation |
|---|---|---|
| A | 2x2.5 | 30* |
| B | 2x1<br>2x12.5 | <br>41.4**<br>100** |
| C | 2x1<br>2x12.5 | 53.3**<br>100** |
| Statistical significance | | |

* p<0.05
** p<0.01

2. Effect of test compound on oxygen tension ($pO_2$) of rat cortex.

A) Animal Preparation.

Male rats (280 – 350g) were anaesthetized with sodium – thio pentone (100 mg/kg i.p.). The arterial blood pressure was recorded from a femoral artery.

B) Measurement.

The cranium was opened with a trephine (0.6 mm) above the praecentral cortex. The dura mata was carefully removed and a multiwire oxygen sensitive electrode was placed on the cortex.

The electrode current was continuously recorded.

After a stabilisation period the test compound was administered. The change in the cortical $pO_2$, produced by compound administration, was calculated for each animal.

These measurements were performed in normal animals and in animals in which both carotid arteries had been ligated 3 weeks previously in order to induce cortical ischaemia.

C) Results.

The results for Compound A are shown in Table 2.

TABLE 2

| Dosage mg/kg i.v. | N | Increase in $_pO_2$ (torr) | Ischaemic Cortex Increase in $_pO_2$ (torr) (mean ± sem) |
|---|---|---|---|
| 0.4 | 3 | 2.1 ± 2.0 | |
| 0.5 | 5 | | 0.9 ± 0.9 |
| 0.8 | 2 | 4.1 ± 0.9 | |
| 1.0 | 5 | | 1.9 ± 1.9 |

The above results show that Compound A increases $_pO_2$ in the rat normoxic and chronic ischaemic cortex.

All results in Table 2 are statistically significant ($p<0.05$)

Example 1

6 – Bromo – 2,2 – dimethyl – 4 – (2 – oxo – 1 – pyrrolidinyl) – 1,2 – dihydroquinoline (Compound B)

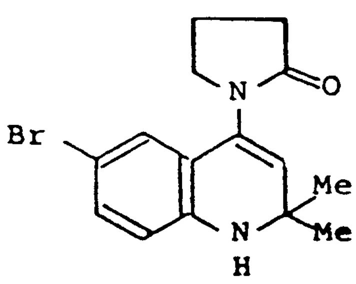

During certain runs of the preparation of trans – 2,2 – dimethyl – 6 – bromo – 4 – (2 – oxo – 1 – pyrrolidinyl) – 1,2,3,4 – tetrahydroquinolin – 3 – ol (as described in Example 3 of WO 85/00602) investigation of the earlier chromatographic fractions in the purification of that compound, revealed the presence of cis – 6 – bromo – 2, 2 – dimethyl – 4 – (2 – oxo – 1 – pyrrolidinyl) – 1,2,3,4 – tetrahydroquinolin – 3 – ol, and 6 – bromo – 2,2 – dimethyl – 4 – (2 – oxo – 1 – pyrrolidinyl) – 1,2 – dihydroquinoline (compound B) which was obtained as crystals from ethyl acetate – pentane: m.p. 177 – 178°C.
NMR ($CDCl_3$) δ
1.32 (s, 6H)

1.93 – 2.70 (series of m, 4H)
3.50 (t, J = 7Hz, 2H)
3.87 (br m, 1H)
5.37 (s, 1H)
6.23 (d, J = 8Hz, 1H)
6.73 (d, J = 2Hz, 1H)
6.93 (q, J = 8, 2Hz, 1H)

Example 2

6 – Cyano – 2,2 – dimethyl – 4 – (2 – oxo – 1 – pyrrolidinyl) – 1,2 – dihydroquinoline (Compound C)

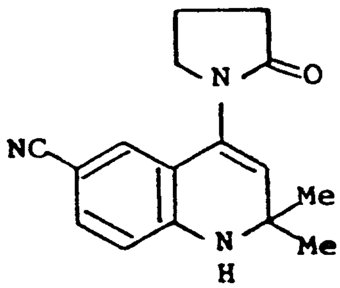

(E2)

6 – Bromo – 2,2 – dimethyl – 4 – (2 – oxo – 1 – pyrrolidinyl) – 1,2 – dihydroquinoline (0.50g) and copper (1) cyanide were heated under reflux in N – methylpyrrolidone (15ml) for 4 hours. The mixture was poured into 30% aqueous ethylenediamine (25ml) and extracted with ethyl acetate. The organic phase was washed with aqueous ethylenediamine, water and brine and dried over anhydrous magnesium sulphate. Filtration and evaporation gave a gum (0.40g) which was combined with the crude product (0.23g) of another run and chromatographed (chromatotron; 30% ethyl acetate – pentane ethyl acetate in a gradient elution). Fractions containing the desired material were combined and recrystallised from ethyl acetate – pentane as a yellow solid (0.19g) of m.p. 207 – 209°C.

NMR ($CDCl_3$) $\delta$
1.39 (s, 6H)
2.11 – 2.66 (m, 4H)
3.60 (t, 7Hz, 2H)
4.00 (br m, 1H)
5.50 (s, 1H)
6.50 (d, J = 9, 1H)
7.01 (d, J = 2, 1H)
7.21 (q, J = 9,2Hz, 1H)

## Claims

**1.** The use of a compound of formula (I):

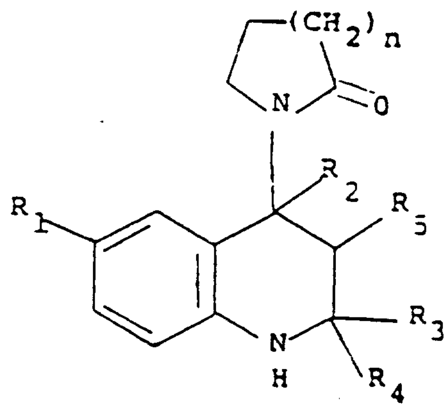

(I)

wherein:
n is 1 or 2;
$R_1$ is chloro, bromo, $C_{1-6}$alkylcarbonyl or cyano;
one of $R_3$ and $R_4$ is hydrogen or $C_{1-4}$ alkyl and the other is $C_{1-4}$ alkyl or $R_3$ and $R_4$ together are $C_{2-5}$ polymethylene;
and either
$R_5$ is hydroxy, $C_{1-3}$ alkoxy or $C_{1-8}$ acyloxy; and
$R_2$ is hydrogen and the cyclic amide moiety and $R_5$ are trans; or $R_5$ and $R_2$ together form a bond, for the manufacture of a medicament for the treatment or prophylaxis of cerebrovascular disorders and/or disorders associated with cerebral senility.

**2.** A use according to claim 1, wherein n is 1.

**3.** A use according to claim 1 or 2, wherein $R_1$ is chloro, bromo or cyano.

**4.** A use according to any preceding claim, wherein $R_3$ and $R_4$ are both methyl.

**5.** A use according to any preceding claim, wherein $R_2$ and $R_3$ together form a bond.

**6.** A use according to any of claims 1 to 4, wherein $R_2$ is hydrogen and $R_5$ is hydroxy.

**7.** A use according to claim 1 wherein the compound of formula (I) is 6-bromo-2,2-dimethyl-4-(2-oxo-1-pyrrolidinyl)-1,2-dihydroquinoline,
6-cyano-2,2-dimethyl-4-(2-oxo-1-pyrrolidinyl)-1,2-dihydroquinoline,
trans-2,2-dimethyl-6-cyano-4-(2-oxo-1-pyrrolidinyl)-1,2,3,4-tetrahydro- quinolin-3-ol,
trans-2,2-dimethyl-6-chloro-4-(2-oxo-1-pyrrolidinyl)-1,2,3,4-tetrahydro- quinolin-3-ol,
6-chloro-2,2-dimethyl-4-(2-oxo-1-pyrrolidinyl)-1,2-dihydroquinoline or
trans-2,2-dimethyl-6-bromo-4-(2-oxo-1-pyrrolidinyl)-1,2,3,4-tetrahydro-quinolin-3-ol.

## Patentansprüche

1. Verwendung einer Verbindung der Formel (I)

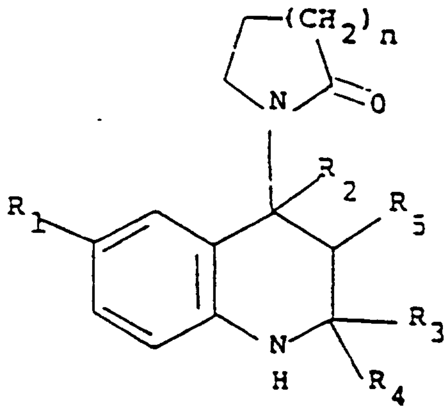

(I)

in der
n 1 oder 2 ist,
$R_1$ ein Chloratom, Bromatom, ein $C_{1-6}$-Alkylcarbonyl- oder Cyanorest ist,
einer der Reste $R_3$ und $R_4$ ein Wasserstoffatom oder ein $C_{1-4}$-Alkylrest ist und der andere ein $C_{1-4}$-Alkylrest ist oder $R_3$ und $R_4$ zusammen einen $C_{2-5}$-Polymethylenrest bilden, und entweder
$R_5$ ein Hydroxy-, $C_{1-3}$-Alkoxy- oder $C_{1-8}$-Acyloxyrest ist und
$R_2$ ein Wasserstoffatom ist und der cyclische Amidrest und der Rest $R_5$ trans stehen, oder $R_5$ und $R_2$ zusammen eine Bindung bilden, zur Herstellung eines Arzneimittels zur Behandlung oder Prophylaxe cerebrovaskulärer Störungen und/oder Störungen, die mit cerebraler Senilität einhergeben.

2. Verwendung nach Anspruch 1, woben n 1 ist.

3. Verwendung nach Anspruch 1 oder 2, wobei $R_1$ ein Chloratom, Bromatom oder eine Cyanogruppe ist.

4. Verwendung nach einem der vorhergehenden Ansprüche, in der $R_2$ und $R_4$ beide eine Methylgruppe bedeuten.

5. Verwendung nach einem der vorhergehenden Ansprüche, wobei $R_2$ und $R_3$ zusammen eine Bindung bilden.

6. Verwendung nach einem der Ansprüche 1 bis 4, wobei $R_2$ ein Wasserstoffatom ist und $R_5$ eine Hydroxygruppe ist.

7. Verwendung nach Anspruch 1, wobei die Verbindung der Formel (I) 6-Brom-2,2-dimethyl-4-(2-oxo-1-pyrrolidinyl)-1,2-dihydrochinolin,
6-Cyano-2,2-dimethyl-4-(2-oxo-1-pyrrolidinyl)-1,2-dihydrochinolin,
trans-2,2-Dimethyl-6-cyano-4-(2-oxo-1-pyrrolidinyl)-1,2,3,4-tetrahydro-chinolin-3-ol,
trans-2,2-Dimethyl-6-chlor-4-(2-oxo-1-pyrrolidinyl)-1,2,3,4-tetrahydro-chinolin-3-ol,
6-Chlor-2,2-dimethyl-4-(2-oxo-1-pyrrolidinyl)-1,2-dihydrochinolinoder
trans-2,2-Dimethyl-6-brom-4-(2-oxo-1-pyrrolidinyl)-1,2,3,4-tetrahydro-chinolin-3-ol ist.

## Revendications

1. Utilisation d'un composé de formule (I) :

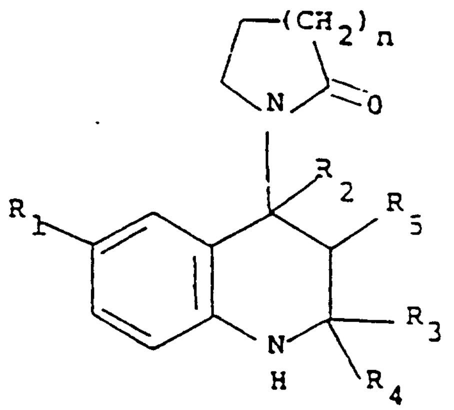

(I)

dans laquelle

n est 1 ou 2 ;

$R_1$ est un atome de chlore, de brome, un groupe alkyl($C_{1-6}$)-carbonyle ou cyano ;

l'un des $R_3$ et $R_4$ est un atome d'hydrogène ou un groupe alkyle en $C_{1-4}$ et l'autre est un groupe alkyle en $C_{1-4}$ ou bien $R_3$ et $R_4$ forment ensemble un radical polyméthylène en $C_{2-5}$ ;

et soit

$R_5$ est un groupe hydroxy, alcoxy en $C_{1-3}$ ou acyloxy en $C_{1-8}$ ; et

$R_2$ est un atome d'hydrogène et la partie amide cyclique et $R_5$ sont trans ; soit $R_5$ et $R_2$ forment ensemble une liaison,

pour la fabrication d'un médicament pour le traitement ou la prophylaxie de maladies cérébrovasculaires et/ou de troubles associés à la sénilité cérébrale.

2. Utilisation suivant la revendication 1, dans laquelle n est 1.

3. Utilisation suivant la revendication 1 ou 2, dans laquelle $R_1$ est un atome de chlore, de brome ou un groupe cyano.

4. Utilisation suivant l'une quelconque des revendications précédentes, dans laquelle $R_3$ et $R_4$ sont tous deux un groupe méthyle.

5. Utilisation suivant l'une quelconque des revendications précédentes, dans laquelle $R_2$ et $R_3$ forment ensemble une liaison.

6. Utilisation suivant l'une quelconque des revendications 1 à 4, dans laquelle $R_2$ est un atome d'hydrogène et $R_5$ est un groupe hydroxy.

7. Utilisation suivant la revendication 1, dans laquelle le composé de formule (I) est :
la 6-bromo-2,2-diméthyl-4-(2-oxo-1-pyrrolidinyl)-1,2-dihydroquinoléine,
la 6-cyano-2,2-diméthyl-4-(2-oxo-1-pyrrolidinyl)-1,2-dihydroquinoléine,
le trans-2,2-diméthyl-6-cyano-4-(2-oxo-1-pyrrolidinyl)-1,2,3,4-tétrahydroquinoléin-3-ol,
le trans-2,2-diméthyl-6-chloro-4-(2-oxo-1-pyrrolidinyl)-1,2,3,4-tétrahydroquinoléin-3-ol,
la 6-chloro-2,2-diméthyl-4-(2-oxo-1-pyrrolidinyl)-1,2-dihydroquinoléine, ou
le trans-2,2-diméthyl-6-bromo-4-(2-oxo-1-pyrrolidinyl)-1,2,3,4-tétrahydroquinoléin-3-ol.